(19) Europäisches Patentamt
European Patent Office
Office européen des brevets

(11) Publication number: **0 174 093 B1**

(12) **EUROPEAN PATENT SPECIFICATION**

(45) Date of publication of patent specification: **18.10.89**

(21) Application number: **85305476.5**

(22) Date of filing: **31.07.85**

(51) Int. Cl.[4]: **C 07 B 33/00,** C 07 C 47/00, C 07 C 49/00, C 07 C 15/04, C 07 C 5/42, C 07 C 45/29, G 01 N 21/76, B 01 J 19/00, B 01 J 23/52

(54) **Method for selective conversion of organic compounds and detecting same by gas chromatography and chemiluminescence detection.**

(30) Priority: **03.08.84 US 637505**

(43) Date of publication of application:
**12.03.86 Bulletin 86/11**

(45) Publication of the grant of the patent:
**18.10.89 Bulletin 89/42**

(84) Designated Contracting States:
**AT BE CH DE FR GB IT LI LU NL SE**

(56) References cited:
**EP-A-0 107 923**
**FR-A-2 285 171**
**FR-A-2 343 505**
**GB-A-2 001 621**
**US-A-3 919 397**
**US-A-4 066 409**

(73) Proprietor: **Sievers Research, Inc.**
**2905 Center Green Court South Suite B**
**Boulder Colorado 80301 (US)**

(72) Inventor: **Sievers, Robert Eugene**
**655 North Star Court**
**Boulder Colorado 80302 (US)**
Inventor: **Nyarady, Stefan Alan**
**4231 Piedra Place**
**Boulder Colorado 80301 (US)**

(74) Representative: **Spence, Anne et al**
**Spence & Townsend Mill House Wandle Road**
**Beddington Croydon Surrey CR0 4SD (GB)**

**EP 0 174 093 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Technical field

This invention relates to novel and improved reduction/oxidation or redox reaction processes involving the catalyzed reduction of oxides of nitrogen, and further relates to a method and apparatus for the selective detection of constituents resulting from such redox reaction processes.

Background art

Numerous processes have been devised for converting certain nitrogen compounds to nitric oxide (NO). Representative of such processes is that disclosed in U.S. Letters Patent No. 3,919,397 to R. K. Gould in which a gas is passed through a plurality of alumina tubes containing wires of a catalytic material, such as, platinum. The wires are connected to a source of electrical energy for resistively heating the wire in order to speed up the thermal conversion of nitrogen to nitric oxide when passed into contact with the wires. In this and other processes, it is known also that one can detect or measure the concentration of nitric oxide by combining ozone with the nitric oxide to produce nitrogen dioxide ($NO_2$) in an excited electronic state, oxygen and subsequently emmited light. The quantity of light produced is a measurement of the concentration or amount of nitric oxide present and typically is carried out by various types of chemiluminescent detectors. Gould, however, suggests that materials, such as gold, which he asserts are consumed in the reaction with $NO_2$ to produce NO are not properly catalysts. Gould fails to recognize the potential of utilizing gold as a catalyst in redox reaction processes in which certain reducing agents when combined with oxides of nitrogen are capable of being converted to more valuable materials.

Numerous other patents and publications may be found in the literature which disclose a variety of approaches to so-called $NO_X$ converters as well as the use of gas or liquid chromatography and chemiluminescence detectors for measuring resultant products of the reaction. Representative patents are U.S. Letters Patent Nos: 4,301,114 to D. P. Rounbehler et al, 4,066,409 to D. H. Fine, 3,963,928 and 3,882,028 to W. J. Zolner, and 4,193,963 to W. Bruening et al. Roundbehler et al, for example, is concerned more with a particular form of molecular sieve for trapping species other than NO and $NO_2$ that could interfere with the detection process; and in Fine, organic nitrogen-containing compounds are chromatographically separated, pyrolyzed in contact with oxygen and the nitric oxide emitted from the decomposition is measured.

Other patents pertain to methods and means for catalytic reduction of oxides of nitrogen. For example, U.S. Letters Patent No: 4,162,235 to G. J. K Acres et al is directed to a catalytic purification process for exhaust gas in which platinum group metals including gold are associated with one or more base metals to carry out catalytic reduction of oxides of nitrogen in the presence of suitable reducing agents. In U.S. Letters Patent No: 4,028,274 to H. R. Kunz, various metal catalysts are proposed for use in combination with a support material, such as carbon powder and broadly suggest the utilization of gold as a catalyst but not in any specific reaction or process.

U.K. Patent Application GB 2001621A teaches a method of producing chemicals without the use of catalysts. The method employs liquid $N_2O_4$ as the oxidizing agent, and the preferred reaction temperature is between 0°C and 20°C. In contrast, the present invention employs a gold metal catalyst. Further, the present invention is heterogeneous, occurring at a gold surface, while the above application requires no solid surface or catalyst. Although the present invention employs liquid $N_2O_4$ confined in a Teflon (Trade Name) permeation tube, the liquid never can contact the organic chemicals being oxidized. The $N_2O_4$ first becomes dissociated to $NO_2$ and passes through the Teflon wall of the permeation tube, where it is diluted in a carrier gas before being contacted with the compounds to be oxidized. The temperature range of the present invention is much higher, from 200°C to 400°C, which means the reactants in the Application GB—A—2001621 are cooled to slow down the reaction, while the present invention heats the reactants to accelerate this reaction. The present reaction requires the formation of nitric oxide while the above Application does not.

To the best of our knowledge, no one has recognized the ability to carry out a redox reaction, i.e. a reduction oxidation process in which oxides of nitrogen are combined with a reducing agent, such as, alcohols, olefins, aldehydes or other selected organic compounds which can be catalyzed by a heated gold surface to convert the organic compounds selected to new, more valuable, oxidized or dehydrogenated species. A related feature of the present invention resides in the ability to selectively detect the new species by a combination of gas chromatography and chemiluminescent measurement without detecting the presence of other compounds, such as alkanes, chlorinated hydrocarbons, water, oxygen and nitrogen. Since oxygenated organic compounds typically exist at trace levels in the atmossphere while the alkanes are present at much higher concentrations, this selective process is useful in improving the detection of the oxygenated constituents of the atmosphere as well as in other complex matrices, such as, petroleum and fossil fuel products, beverages, and fragrances.

Disclosure of invention

In accordance with the present invention, there is provided in one aspect a process for the detection of organic compounds comprising:

catalyzing a reduction-oxidation reaction between a nitrogen oxide oxidizing agent, selected from

nitric acid and nitrogen dioxide, and a compound capable of reducing said oxidizing agent to nitric oxide, said reducing compound being selected from alcohols, aldehdyes, amines, ketones, phenols, olefins, aromatic compounds, and oxygen, sulphur and nitrogen-containing compounds of the foregoing and mixtures thereof, characterised in that the reaction is in the presence of a solid elemental gold catalyst heated to a temperature in the range 200° to 400°, whereby said reducing compound is oxidized to a more highly unsaturated, dehydrogenated oxidation state and nitric oxide is thereby produced; and characterised by

contacting said produced nitric oxide with ozone, whereby chemiluminescence is emitted; and

detecting the emitted chemiluminescence whereby the measure of light provides an indication of the amount of said reducing compound.

In another aspect the invention provides a method of synthesis of organic compounds by catalyzed oxidation of organic reducing compounds selected from alcohols, aldehydes, amines, ketones, phenols, olefins, aromatic compounds and oxygen, sulphur and nitrogen-containing compounds of the foregoing and mixtures thereof characterized in that:

the organic reducing compound is contacted with nitrogen dioxide over a catalyst of supported elemental gold metal, under conditions of elevated temperature in the range 200°C to 400°C such that the nitrogen dioxide is reduced to nitric oxide and the organic reducing compound is dehydrogenated to form a product of increased oxidation state; and

said dehydrogenated organic reducing compound product is recovered.

According to a further aspect of the invention, a redox reactor apparatus has been devised, wherein a chromatographic column is arranged to receive organic redox reactor apparatus has been devised, wherein a chromatographic column is arranged to receive organic compounds. A conducting means leads from an output end of the column and is connected to a means defining a source of oxides of nitrogen. A further means introduces selected oxides of nitrogen with an inert carrier through the source of oxides of nitrogen and into the conducting means. A reaction chamber is connected to the conducting means for receipt of the organic compounds, oxides of nitrogen, and inert carrier therethrough and includes a catalyst bed composed of elemental gold metal. The reaction chamber also includes a heating means for maintaining the gold catalyst at an elevated temperature sufficient to reduce any oxides of nitrogen present to nitric oxide and to convert organic compounds present to higher oxidized forms.

The method and apparatus have useful application to the detection of species which appear in the atmosphere as well as in other complex matrices, such as, petroleum and fossil fuel products, beverages and fragrances, and also for the recovery of higher oxidized forms or species resulting from the group consisting of reducing agents, including alcohols, aldehydes, amines, ketones, olefins, and aromatic compounds.

Brief description of drawings

The details of this invention will be described in connection with the accompanying drawings, in which:

Figure 1 is a schematic diagram of a preferred form of detector apparatus constructed in accordance with the present invention; and

Figure 2 illustrates representations of chromatograms produced by the detectors in accordance with Example 5.

Detailed description

There is shown by way of illustrative example in Figure 1 of this application a preferred apparatus for the selective detection of constituents after elution from a chromatographic column apparatus represented at 10 and having an injection port 11. The constituents are directed through a main conduit 12 for mixture with nitrogen dioxide in a gas carrier which, for example, may be helium or air from a carrier gas source 13 as is directed into the main conduit 12 via permeation tube 14 containing a source of $NO_2$. The resultant mixture in the conduit 12 is directed into the inlet side of a quartz tube 15 in which is contained a gold catalyst bed as represented at 16. This bed is heated by suitable means 17 to an elevated temperature on the order of 200°C. to 400°C. and may be monitored with a thermocouple, not shown. The constituents which are introduced via the column 10 are typically reducing agents, such as, alcohols, aldehydes, amines, ketones, olefins and aromatic compounds which, when mixed with nitrogen dioxide and passed through the confined area containing the heated catalyst bed 16 will reduce the nitrogen dioxide to nitric oxide.

The apparatus 10 may, for purposes of illustration, be an F&M model (Trade name) 810 gas chromatograph, having a 1/4 inch (0.635 cm) by 5 foot (1.524 m) glass column packed with 0.1% SP-1000 on Carbopack C (Trade name), manufactured and sold by Supelco, Inc., of Bellefonte, Pennsylvania, United States, or a fused silica capillary column (HP-Ultra (Trade name), 20 meters long, 0.3 mm inner diameter, coated with cross-linked polydimethylsiloxane, film thickness 0.5 µm, manufactured and sold by Hewlett-Packard Company, Avondale Pennsylvania, United States. Retention times of compounds may be established using a flame ionization detector or other conventional detectors. When the ionization detector is removed, the redox detector is connected via a glass or Teflon (Trade name) transfer line which is installed between the column 10 and the $NO_2$ makeup gas tee from the tube 14. This transfer line may be resistively heated to a temperature on the order of 50°C. and may introduce up to on the order of 6 ml of

dead volume. The flow of the carrier gas and entrained $NO_2$ may, for example, be maintained at 25 ml per minute. Concentrations of $NO_2$ in the make-up gas can be varied by changing the temperature of the porous Teflon (Trade name) permeation tube 14 which acts as a reservoir for liqiud $NO_2$. Typically, a few parts per million of $NO_2$ in the make-up gas stream is more than sufficient to oxidize the organic compound in the effluent from the chromatographic column. The chromatographic column 10 is useful in the present invention for the purpose of rapidly separating those volatile compounds in a mixture which will be oxidized in reducing the nitrogen dioxide to nitric oxide.

For instance, the following compounds or elements do not produce NO: water, carbon dioxide, oxygen, helium, nitrogen, argon, dichloromethane, acetonitrile, n-pentane, n-hexane, n-decane, 1,2-dichlorethane, cyclohexane, chloroform, and tetrachloroethane. The following compounds or elements do produce NO and therefore are selectively detected by this redox system: hydrogen, acetone, 2-butanone, 4-methyl-2-pentanone, methanol, ethanol, n-propanol, n-butanol, formaldehyde, acetaldehyde, propanol, n-butanal, n-pentanal, n-hexanal, formic acid, acetic acid, n-propanoic acid, n-butanoic acid, benzene, toluene, benzaldehyde, hydrogen peroxide, 2-hexene, n-butyl ether, cyclohexene 1 - methylcyclohexene, ammonia and carbon monoxide.

Solutions of hexanal in dichloromethane and a five component mixture of n-butanal, ethyl acetate, n-butanol, benzene and 4 - methyl - 2 - pentanone in dichloromethane were injected into the gas chromatographic system, separated by the gas chromatographic column and detected by the catalytic redox production of NO from $NO_2$. Ethyl acetate represents an intermediate class of compounds which only partially react under the conditions typically used (temperature, flow rate, etc.), the partial reaction (~1—10%) generating a response at least an order of magnitude less than the other responsive compounds. Compound concentrations ranged from 0.5 µg/µl to 2 µg/µl, and 0.4 µl to 1.0 µl of solution were injected.

Linearly extrapolated detection limits for the packed column system described herein are approximately 40 ng of ethanol, methanol, benzene, 4 - methyl - 2 - pentanone, and hexanal, and 300 ng of n-butanal. Using capillary columns and reducing the system dead volume has improved the detection limit by more than an order of magnitude. Using a capillary column creates narrower compound peaks, which also allows more sensitive detection. For example, with a capillary column 10, 0.1 ng of n-octanol, n-decanol, 2,6 - dimethylphenol and 2,6 - dimethylaniline can be detected.

At the catalyst bed, one suitable form of supported gold is formed by 10 grams of 1/2 mm diameter borosilicate glass beads introduced to an aqueous solution of 0.1 gram $HAuCl_4 \cdot 3H_2O$. This solution is evaporated to dryness, then further dried at 110°C. for two hours at $0.66\times10^{-3}$ (0.5 torr). The gold coated beads are then reduced to elemental gold under a flowing $H_2$ atmosphere at 300°C. for two hours. Two grams of gold coated glass beads 16 are then placed in a 6 mm diameter quartz tube 15 with silanized glass wool plugs to form the 10 cm long active catalyst bed. The catalyst is heated resistively to 300°C. and the temperature monitored with a thermocouple.

For the purpose of illustration, a typical gas handling system may consist of bottled air from a gas carrier source 19 which is passed through a series of traps to insure a minimum amount of impurities. As shown, a 13X molecular sieve trap 20 is positioned in inlet line 21 to remove water vapor and a Hopcalite (Trade name) chamber 22 serves to remove carbon monoxide. An in-line UV ozonizer 23 creates ozone to oxidize NO to $NO_2$ in a 500 mL holdup reaction volume 24. $NO_2$ and $CO_2$ are removed by an Ascarite (Trade name) trap 25, and residual ozone may be scrubbed by activated charcoal, not shown. A final 4A molecular sieve trap 26 is used to remove $SO_2$ and hydrocarbons, thereby to assure the supply of high purity air or helium for the entire system, including the nitrogen dioxide permeation tube chamber 14 via a split tee 13.

A nitric oxide analyzer takes the form of a chemiluminescence detector 30, which for purposes of illustration may be a Thermo Electron Corporation Model No. 14D dual channel chemiluminescence $NO/NO_x$ analyzer. The analyzer is modified by installation of a valve to isolate the molybdenum $NO_x$ catalyst, allowing the instrument to serve as a single channel NO detector without depleting the molybdenum catalyst. Flow through the instrument reaction chamber as represented at 32 is maintained by a metal bellows pump 38 and regulated by a flow restrictor capillary, not shown, which also serves to reduce the pressure in the chamber 32 for improved light emission. An internal electric discharge ozone generator as represented at 34 directs ozone through a capillary restrictor, not shown, to the reaction chamber 32, which is mounted directly in front of the photomultiplier tube 36. The photomultiplier tube 36 detects the chemiluminescence emitted from the $NO_2$ that is produced in an excited electronic state, or $NO_2^*$, by the subsequent reaction of ozone with the NO produced from the reactions in the heated catalyst bed. Then upon relaxation of the $NO_2$ from an excited state, according to the reaction scheme:

$$NO+O_3 \rightarrow NO_2^* \rightarrow NO_2+Hv,$$

light is emitted which can be measured continuously with a photomultiplier tube and photon counting techniques. Higher oxidized species that are formed as a result of the reduction of $NO_2$ are recovered through a cold trap designated at 37.

The following examples are presented by way of illustration and not limitation for a further understanding of the features and aims of the present invention:

Example 1

Alcohols were converted to aldehydes by reaction with nitrogen dioxide at gold surfaces heated to approximately 300°C. A gold catalyst bed was prepared which consisted of glass beads upon which gold was coated from gold chloride aqueous solutions by already well-established techniques. For example, ten grams of borosilicate glass beads approximately 0.5 mm in diameter was added to an aqueous solution containing 0.1 gram of $HAuCl_4 \cdot 3H_2O$. The solution was evaporated to dryness, after which the beads were further dried at 110° for two hours at $0.66\times10^{-3}$ Barr (0.5 torr). The gold salt-covered beads were then treated by heating at 300°C. for 2 hours under a flowing hydrogen stream to form a layer of elemental gold on the surfaces of the glass beads. Two grams of gold coated beads were then placed in a quartz tube with a 6 mm outer diameter and a 4 mm inner diameter. The beads were held in place in the tube to form a 10 cm long catalyst bed with small porous end plugs made of silanized glass wool. The catalyst bed is heated typically at temperatures between 200°C. and 400°C., and the temperature is maintained constant at an optimal value determined by considerations of the rates of the reaction which, as expected, are faster at higher temperatures, vs. energy consumption and thermal stability of reactants and products, the latter factors favoring the use of lower temperatures.

A carrier gas which does not participate in the reaction was used to move reactants and products through the catalyst bed in one-pass tests. Helium is generally used for analytical applications, but nitrogen or air or the reactants themselves also can be used as a carrier to facilitate transport. In this example, bottled compressed air was first passed through a trap containing Linde (Trade name) 13× molecular sieves to remove water and other impurities, then through a Hopcalite (Trade name) trap to remove carbon monoxide. An in-line ultraviolet light ozone generator caused oxidation of NO to $NO_2$. Any $NO_2$ and $CO_2$ present were then removed by passing the air stream through an Ascarite (Trade name) trap. Residual ozone was removed by a bed of activated charcoal. A final bed of Linde (Trade name) 4A molecular sieve was used to remove $SO_2$ and any other organic compound impurities remaining in the air stream.

This purified air stream, with a flow-rate of 10 standard cubic centimeters per minute, then was passed through a thermostated chamber (25°C.) with a Teflon (Trade name) permeation tube containing nitrogen dioxide and its dimer. The permeation tube was a porous Teflon (Trade name) polymer sealed at both ends and, at 25°C., was calibrated gravimetrically to have an emission rate of 10.2 µg/min of $NO_2$.

Flow through the system was maintained by a metal bellows pump at the outlet and a flow restrictor glass capillary (0.5 mm i.d.) at the exit of the catalyst bed. Total augmented air flow was one L/min when the pressure in the catalyst bed was 0.84 Bar (630 torr). The calculated linear velocity through the bed was 130 cm/sec, so the mean residence contact time in the catalyst bed was less than 0.1 sec. Longer residence times can be used at some sacrifice in throughput, but even with these short catalyst contact times conversion of several compounds was accomplished with high yields (greater than 90%). When 0.37 µg of 1 - pentanol was injected into the carrier gas stream entering the catalyst bed heated at 399°C., the products were collected with a Tenax (Trade name) polymeric sorbent. Thermal desorption of the sorbed samples with cryogenic focusing at the inlet of a capillary chromatography column and subsequent gas chromatographic/mass spectrometric analysis revealed that no unreacted 1-pentanol was detected and that the alcohol was converted in excellent yield to the aldehyde, 1-pentanal. Nitric oxide was formed as the expected product from reducing nitrogen dioxide. The formation of nitric oxide was confirmed by chemiluminscence measurement upon reaction with ozone, as previously described.

Example 2

In the same apparatus, with similar conditions to those described in Example 1, the gold-catalyzed redox process involving the reaction of 4 - methyl - 2 - pentanol was injected and the reaction products analyzed after a single pass through the reactor, the only oxidation product that could be detected by the gas chromatography/mass spectrometry was the ketone, 4 - methyl - 2 - pentanone. In Example 1, a primary alcohol was converted to the corresponding aldehyde, while this Example shows that secondary alcohols are oxidized to form the corresponding ketone by the following reaction:

$$CH_3-\underset{|}{\overset{OH}{CH}}-CH_2-\overset{CH_3}{\overset{|}{CH}}-CH_3+NO_2 \xrightarrow[399°C.]{Au} CH_3-\overset{O}{\overset{\|}{C}}-CH_2-\overset{CH_3}{\overset{|}{CH}}-CH_3+NO+H_2O$$

The process has utility in both synthesis and analysis. When a ketone is desired from a secondary alcohol, the above reaction can be conducted. If desired, the NO formed as a reaction product can be reconverted to $NO_2$ by reaction with oxygen or air at an elevated temperature and recycled.

To be useful analytically as a sensor or chromatography detector, the novel process can be combined by the well-establishzd reaction with ozone to constitute a new selective detection system. For example, an alcohol eluted from a chromatographic column can be passed through the catalyst bed to form NO in the reaction described above. NO can be detected by chemiluminescence upon mixing with ozone as described above.

Example 3

Another experiment, using the same apparatus and conditions similar to the above examples, was performed using cyclohexene as the starting material. In this instance, the catalyzed dehydrogenation process yielded benzene. Unlike the earlier two examples, however, there was a substantial amount of the starting material left after the single-pass reaction process, under the flow, temperature and concentration conditions used. This experiment demonstrated that olefins can be dehydrogenated by $NO_2$ catalytically to form less saturated products, and that olefins can be detected by chemiluminescence measurement of the NO formed.

Example 4

With the same apparatus and under nearly the same conditions detailed in Example 1, we studied the gold-catalyzed reaction of a ketone, 4 - methyl - 2 - pentanone, with nitrogen dioxide. After one pass of a 2.5 µg sample of 4 - methyl - 2 - pentanone, a small amount of 4 - methyl - 3 - pentene - 2 - one was formed, giving rise to about 3% of the total ion chromatogram of the effluent from the catalyst bed. Most of the effluent was identified as unreacted starting material. This experiment demonstrated that ketones are dehydrogenated by $NO_2$ catalytically to form unsaturated ketones, and that ketones can be detected by chemiluminescent measurement of the NO formed.

Example 5

Another example of the invention represents a preferred embodiment for use as a chromatographic detector for analysis of mixtures of compounds:

Small glass beads were used as the catalyst bed. One gram of 0.064—0.044 mm (230—325 mesh) borosilicate glass beads was added to an aqueous solution of 0.5 grams of $HAuCl_4 \cdot 3H_2O$. These beads were further treated in a manner similar to that in Example 1. Fifteen milligrams of gold-coated glass beads were placed in a glass tube of 1.8 mm inner diameter and 6 mm outer diameter. The catalyst bed was 0.7 cm long and was heated as in Example 1. The chromatographic system used was an F&M Model 810 (Trade name) gas chromatograph, equipped with a 20 meter long by 0.31 mm diameter fused silica capillary column, with a 0.52 µm film thickness of cross-linked polydimethylsiloxane manufactured by Hewlett-Packard Company of Avondale, Pennsylvania.

Since this is an analytical separation application, a closed gas flow system, consisting of purified helium, was used to carry $NO_2$ from the permeation tube into the catalytic reduction chamber. Helium was also used as the chromatographic carrier gas, at a flow rate of approximately one milliliter per minute. A separate helium source supplied 30 to 150 milliliters per minute, at 68.9 kPa (10 psig), to the nitrogen dioxide permeation device described in Example 1. This nitrogen dioxide-doped helium flow was added to the effluent of the gas chromatographic column just prior to its entering the heated catalyst bed. A minimum length of Teflon (Trade name) tubing, with a volume of 1 milliliter or less, connected the exit of the catalyst bed directly to the chemiluminenscence reaction chamber.

When a mixture of approximately twenty nanograms each of ten different compounds (n-decane, n-octanol, 2,6 - dimethylphenol, 2,6 - dimethylaniline, napthalene, n-decanol, n - tridecane, methyl decanoate, n-tetradecane and methyl undecanoate) was injected into the gas chromatograph, only five of the compounds were selectively detected using the redox reaction system of this invention. The five compounds, n-octanol, n-decanol, naphthalene, 2,6 - dimethylaniline and 2,6 - dimethylphenol, were oxidized, reducing nitrogen dioxide to nitric oxide, which was detected downstream by chemiluminescence upon mixing with ozone. By contrast, the alkanes (decane, tridecane and tetradecane) gave no response, and the two methyl esters (methyl decanoate and methylundecanoate) gave intermediate responses. If flame ionization detection, all ten compounds will produce responses.

Example 6

Another experiment using the same apparatus and conditions described in Example 5 was performed using a commercially available perfume sample sold under the trademark Esteé Lauder, Private Collection (pure undiluted fragrance). From a comparison of a flame ionization detector chromatogram with that of a chemiluminescent detector as described, the differences in the number of peaks and in relative peak heights illustrated the selective discrimination between different compounds by the chemiluminescent detector.

Example 7

Apparatus similar to that described in Example 5 was modified to divert one-half of the flow exiting the analytical gas chromatographic column and to direct it to a standard flame ionization detector. The remaining half of the gas flow proceeded unaffected through the heated catalyst bed as in Example 5. An auxiliary source of helium, approximately 2 ml per minute, was supplied at the point where the flow was split in order to maintain the same linear gas velocity to both detectors.

Figure 2 illustrates a representative pair of chromatograms produced by this parallel detector arrangement, the bottom trace showing the same ten components identified in Example 5, and the upper trace demonstrating the selective detection achieved. Accordingly, this arrangement permitted direct comparison of components eluted from the chromatographic column in terms of retention time and

relative response for the novel redox chemiluminescent detector versus a standard flame ionization type detector. This example demonstrates the advantage of using the redox detector coupled with other detectors either in parallel or in series. For example, chromatograms produced by this parallel detector arrangement demonstrated that the redox detector is selective and exhibits no chromatographic peak at the retention times of n-decane 40, n-tridecane 41 and n-tetradecane 42, which were detected by the flame ionization detector. Further, the ratios of the peak heights and areas were different for each of the compounds to which the redox detector responded, when compared with the peak heights and areas measured by the flame ionization detector. For instance, the peak areas were nearly the same for n-octanol 43 as measured by either detector, while the peaks drawn from the signals of the redox detector were larger for 2,6 - dimethylphenol 44 and 2,6 - dimethylaniline 45, but smaller for n-decanol 46, than those from the flame ionization detector. The ratios of the carriers can be used to help identify or confirm the identity of unknown compounds by comparing peak area ratios with those from authentic known standard samples. This process of employing parallel detectors, as described, greatly enhanced the power of chromatographic analysis based on retention data.

Examples 3 and 4 demonstrated that, even when there are no easily oxidizable functional groups present, our novel process can yield products of dehydrogenation. However, it appears that the hydrogens being removed must be activated by a functional group, because saturated hydrocarbons such as n-hexane do not react with $NO_2$ at gold surfaces to give any detectable nitric oxide (or presumably any organic oxidation or dehydrogenation products). This forms the basis for the selectivity of sensors and chromatographic detectors based on the novel gold-catalyzed redox reaction process coupled with chemiluminescence following the reaction of nitric oxide with ozone. Under the conditions chosen, no detectable reaction occurs at gold surfaces between nitrogen dioxide and n-alkanes, chlorinated hydrocarbons, nitrogen, oxygen, water, argon, acetonitrile or carbon dioxide. By contrast, compounds that readily can be oxidized or dehydrogenated, such as alcohols, aldehydes, carboxylic acids, phenols, amines, esters, aromatic compounds, ammonia, hydrogen peroxide, carbon disulfide, and hydrogen undergo gold-catalyzed reaction with $NO_2$. These oxidation/dehydrogenation processes are more selective and specific than most presently used processes based upon, for example, reaction with oxygen, and therefore have utility for synthesis. Concurrently, use of this redox process forms the basis for chromatographic detection systems that are more selective than most commonly used detectors, allowing detection of trace species of interest in the presence of much larger concentrations of less interesting or less significant compounds, such as saturated or chlorinated hydrocarbons, water, oxygen and carbon dioxide. Furthermore, our new process makes it possible to detect species such as ammonia and hydrogen, which cannot be measured sensitively with commonly used chromatography detectors such as the hydrogen flame ionization detector.

From the foregoing, it is apparent that substitutions of nitrogen dioxide with nitric acid or other oxides of nitrogen with higher oxidation states than nitric oxide are envisioned as equivalent in the process of catalytic reductions to form NO. Moreover, it is apparent from Example 5 that samples containing high concentrations of large numbers of alkanes could be analyzed for phenols, alcohols, amines and other easily oxidized or dehydrogenated species, with greater efficacy and accuracy and with fewer interferences from the alkanes or other non-reactive compounds, by use of the redox chemiluminescence detection system of this invention as compared with flame ionization detection.

The redox reaction chamber, coupled with a chemiluminescence detector, can be attached to any typical gas chromotographic system in place of any existing detector, or in series with a non-destructive detector. The redox reactor incorporates a heated catalyst bed with provision for adding sufficient make-up gas doped with reactant $NO_2$ gas. While gold surfaces in several different forms can catalyze reaction of $NO_2$ to form NO, the catalyst demonstrated to be most effective consists of elemental gold supported on small glass beads and packed in a hollow quartz tube, as described. Other types of gold surfaces show varying degrees of effectiveness. The novel redox process can also be used as a detector for constituents in the effluents of liquid chromatography or super-critical fluid chromatography columns, if the effluents are contacted with nitrogen dioxide at heated gold surfaces.

**Claims**

1. A process for the detection of organic compounds comprising:

catalyzing a reduction-oxidation reaction between a nitrogen oxide oxidizing agent, selected from nitric acid and nitrogen dioxide, and a compound capable of reducing said oxidizing agent to nitric oxide, said reducing compound being selected from alcohols, aldehydes, amines, ketones, phenols, olefins, aromatic compounds, and oxygen, sulphur and nitrogen-containing compounds of the foregoing and mixtures thereof, characterised in that the reaction is in the presence of a solid elemental gold catalyst heated to a temperature in the range of 200°C to 400°C, whereby said reducing compound is oxidized to a more highly unsaturated, dehydrogenated oxidation state and nitric oxide is thereby produced; and characterised by

contacting said produced nitric oxide with ozone, whereby chemiluminescence is emitted; and

detecting the emitted chemiluminescence whereby the measure of light provides an indication of the amount of said reducing compound.

2. The process of Claim 1 wherein said catalyst is in the form of supported gold metal.

3. The process of Claim 2, wherein said catalyst is in the form of gold coated glass beads placed in a quartz tube.

4. The process of any of Claims 1 to 3, further comprising the step of chromatographically separating the organic compounds prior to said reduction-oxidation reaction.

5. The process of Claim 4, wherein said chromatographic separation is accomplished by a technique selected from gas chromatography, liquid chromatography and super-critical fluid chromatography.

6. The process of Claim 4 or Claim 5, wherein said chromatographic separation is conducted in a continuous flow.

7. The process of any of Claims 1 to 6, wherein said reducing compound and said oxidizing agent react in the vapor phase.

8. The process of any of Claims 1 to 6, wherein said reducing compound and said oxidizing agent react in the liquid phase.

9. A method of synthesis of organic compounds by catalyzed oxidation of organic reducing compounds selected from alcohols, aldehydes, amines, ketones, phenols, olefins, aromatic compounds and oxygen, sulphur, nitrogen containing compounds of the foregoing and mixtures thereof characterized in that:

the organic reducing compound is contacted with nitrogen dioxide over a catalyst of supported elemental gold metal, under conditions of elevated temperature in the range 200°C to 400°C such that the nitrogen dioxide is reduced to nitric oxide and the organic reducing compound is dehydrogenated to form a product of increased oxidation state; and

said dehydrogenated organic reducing compound product is recovered.

10. A method according to Claim 9, wherein said reducing compound is cyclohexene and said product is benzene.

11. A method according to Claim 9, wherein said reducing compound is an olefin and said product is an aromatic compound or a less saturated compound.

12. The method of any of Claims 9 to 11, further characterized in that:

after said mixture of reducing compound and nitrogen dioxide is directed over the gold catalyst, the nitric oxide is oxidized to nitrogen dioxide gas;

the nitrogen dioxide gas from the dehydrogenated organic reducing agent is separated; and

the recovered nitrogen dioxide gas is routed into further incoming organic reducing compound.

13. A redox reactor apparatus for converting organic compounds, comprising in combination:

a chromatographic column (10) into which organic compounds are introduced;

conducting means (12) leading from an output end of said column;

means (14) defining a source of oxides of nitrogen connected to said conducting means (12);

means (13) for introducing selected oxides of nitrogen with an inert carrier through said source (14) of oxides of nitrogen and into said conducting means (12); and

a reactor chamber (15) connected to the conducting means (12) for receipt of organic compounds, oxides of nitrogen and inert carrier therethrough and including a catalyst bed (16) composed of elemental gold metal, including means (17) for heating said gold metal to an elevated temperature sufficient to reduce any oxides of nitrogen present to nitric oxide and to convert organic compounds present to higher oxidized forms.

14. Apparatus in accordance with Claim 13, including chemiluminescent analyzer means (30) for selectively detecting the higher oxidized forms of the organic compounds recovered from said chromatographic column.

15. Apparatus in accordance with Claim 14, wherein said analyzer means (30) includes ozone generator means (34) for producing ozone to undergo reaction with the nitric oxide produced by said catalyst bed (16) whereby to form nitrogen dioxide in an excited electronic state.

**Patentansprüche**

1. Verfahren zum Nachweis organischer Verbindungen durch Katalysieren einer Reduktions-Oxidationsreaktion zwischen einem Stickstoffoxid oxidierenden Mittel aus der Gruppe Salpetersäure und Stickstoffdioxid und einer das oxidierende Mittel zu Stickoxid reduzierenden Verbindung aus der Gruppe der Alkohole, Aldehyde, Amine, Ketone, Phenole, Olefine, aromatischen Verbindungen und Sauerstoff, Schwefel sowie Stickstoff enthaltenden Verbindungen und Mischungen der vorgenannten Verbindungen, dadurch gekennzeichnet, daß die Reaktion unter Anwesenheit eines festen elementaren Gold Katalysators, der auf eine Temperatur im Bereich von 200°C bis 400°C erhitzt wird, durchgeführt wird, wobei die reduzierende Verbindung zu einer höher ungesättigten, dehydrierten Oxidationsstufe oxidiert wird und dabei Stickoxid erzeugt wird, daß das so erzeugte Stickoxid mit Ozon unter Emittieren von Chemolumineszenz in Kontakt gebracht wird, und daß die emittierte Chemolumineszenz detektiert wird, wobei die Messung des Lichtes ein Maß für den Anteil der reduzierenden Verbindung ist.

2. Verfahren nach Anspruch 1, dadurch gekennzeichnet, daß der Katalysator in Form eines Gold-Metall Trägerkatalysators vorliegt.

3. Verfahren nach Anspruch 2, dadurch gekennzeichnet, daß der Katalysator in Form von goldbeschichteten, in einem Quartzrohr untergebrachten Kugeln ausgebildet ist.

4. Verfahren nach einem der Ansprüche 1 bis 3, dadurch gekennzeichnet, daß die organischen Verbindungen vor der Reduktions-Oxidationsreaktion chromatographisch getrennt werden.

5. Verfahren nach Anspruch 4, dadurch gekennzeichnet, daß die chromatographische Trennung durch Gaschromatographie, Flüssigkeitschromatographie und überkritische Fluide Chromatographie vorgenommen wird.

6. Verfahren nach Anspruch 4 oder 5, dadurch gekennzeichnet, daß die chromatographische Trennung im kontinuierlichen Fluß durchgeführt wird.

7. Verfahren nach einem der Ansprüche 1 bis 6, dadurch gekennzeichnet, daß die reduzierende Verbindung und das oxidierende Mittel in der Dampfphase reagieren.

8. Verfahren nach einem der Ansprüche 1 bis 6, dadurch gekennzeichnet, daß die reduzierende Verbindung und das oxidierende Mittel in der flüssigen Phase reagieren.

9. Verfahren zur Synthese organischer Verbindungen durch katalytische Oxidation organischer reduzierender Verbindungen aus der Gruppe der Alkohole, Aldehyde, Amine, Ketone, Phenole, Olefine, aromatischer Verbindungen und Sauerstoff, Schwefel sowie Stickstoffenthaltende Verbindungen und Mischungen der vorgenannten Verbindungen, dadurch gekennzeichnet, daß die organische reduzierende Verbindung über einen Gold-Metall Trägerkatalysator mit Stickstoffdioxid bei erhöhter Temperatur im Bereich von 200 bis 400°C in Kontakt gebracht wird, so daß Stickstoffdioxid zu Stickoxid reduziert und die organische, reduzierende Verbindung zu einem Erzeugnis mit erhöhter Oxidationsstufe dehydriert wird, und daß die dehydrierte organische reduzierende Verbindung rückgewonnen wird.

10. Verfahren nach Anspruch 9, dadurch gekennzeichnet, daß die reduzierende Verbindung Cyclohexan und das Erzeugnis Benzin ist.

11. Verfahren nach Anspruch 9, dadurch gekennzeichnet, daß die reduzierende Verbindung ein Olefin und das Erzeugis eine aromatische oder eine geringer gesättigte Verbindung ist.

12. Verfahren nach einem der Ansprüche 9 bis 11, dadurch gekennzeichnet, daß nach dem Überleiten der Mischung aus reduzierender Verbindung und Stickstoffdioxid über den Gold-Katalysator, das Stickoxid zu Stickstoffdioxidgas oxidiert wird, dieses von dem dehydrierten organischen reduzierenden Mittel getrennt und das rückgewonnene Stickstoffdioxidgas in die weiter zugeführte organische reduzierende Verbindung geleitet wird.

13. Redox-Reaktionsapparat zur Umwandlung organischer Verbindungen mit einer Kombination folgender Merkmale: Einer chromatographischen Säule (10), in die organische Verbindungen aufgegeben werden;

eine an das Ausgangsende der Säule (10) angeschlossene Leitung;

Mittel (14), die eine Stickstoffoxid-Quelle bilden die mit der Leitung verbunden ist;

Mittel (13), zur Zugabe ausgewählter Stickstoffoxide mit einem inerten Träger durch die Stickstoffoxid-Quelle (14) in die Leitung (12);

eine Reaktionskammer (15), die zur Aufnahme der organischen Verbindungen, der Stickstoffoxide und des inerten Trägers mit der Leitung (12) verbunden ist und ein Katalysatorbett (16) aus elementarem metallischem Gold sowie mittel (17) zum Aufheizen des elementaren metallischen Golds auf eine ausreichend erhöhte Temperatur aufweist, um die vorhandenen Stickstoffoxide zu Stickoxid zu reduzieren und die vorhandenen organischen Verbindungen in eine höher oxidierte Form umzusetzen.

14. Apparat nach Anspruch 13, dadurch gekennzeichnet, daß Mittel (30) zur chemolumineszenten Analyse zur selektiven Erfassung der höher oxidierten Formen der organischen Verbindungen, die in der chromatographischen Säule gewonnen werden, vorgesehen sind.

15. Apparat nach Anspruch 14, dadurch gekennzeichnet, daß die Mittel (30) zur Analyse einen Ozongenerator (34) zur Erzeugung von Ozon aufzuweisen, das mit dem durch das Katalysatorbett (16) erzeugten Stickoxid unter Bildung von Stickstoffdioxid in einem angeregten Elektronen-Zustand reagiert.

**Revendications**

1. Procédé de détection de composés organiques du type comprenant la catalyse d'une réaction d'oxydo-réduction entre un agent oxydant du type oxyde d'azote, choisi dans le groupe constitué par: acide nitrique et dioxyde d'azote, et un composé capable de réduire ledit agent oxydant ou oxyde nitrique, ce composé réducteur étant choisi dans le groupe: alcools, aldéhydes, amines, cétones, phénols, oléfines, composés aromatiques et de l'oxygène, du soufre et des composés azotés précités et des mélanges de ces produits, caractérisé en ce que la réaction est effectuée en présence, comme catalyseur, d'or solide élémentaire à température de 200°C à 400°C, le composé réducteur étant oxydé en un état d'oxydation plus fortement insaturé et déshydrogéné, avec production d'oxyde nitrique; et en ce que l'on met en contact l'oxyde nitrique produit avec de l'ozone, ce qui provoque une émission de chimiluminescence, la mesure de la lumière fournie par détection de cette émission donnant alors une indication de la quantité dudit composé réducteur.

2. Procédé selon la revendication 1, caractérisé en ce que ledit catalyseur est sous la forme d'or métallique sur support.

3. Procédé selon la revendication 2, caractérisé en ce que ladite forme consiste en des perles de verre recouvertes d'or, disposées dans un tube en quartz.

4. Procédé selon l'une quelconque des revendications 1 à 3, caractérisé en ce qu'il comporte en outre une étape de séparation chromatographique des composés organiques avant ladite réaction d'oxydo-réduction.

5. Procédé selon la revendication 4, caractérisé en ce que la séparation chromatographique est faite par une technique choisie dans le groupe: chromatographie gazeuse, chromatographie liquide et chromatographie par fluide supercritique.

6. Procédé selon l'une quelconque des revendications 4 à 5, caractérisé en ce que la séparation chromatographique est faite en écoulement continu.

7. Procédé selon l'une quelconque des revendications 1 à 6, caractérisé en ce que ledit composé réducteur et ledit agent oxydant réagissent en phase vapeur.

8. Procédé selon l'une quelconque des revendications 1 à 6, caractérisé en ce que ledit composé réducteur et ledit agent oxydant réagissent en phase liquide.

9. Méthode de synthèse de composés organiques par oxydation catalysée de composés organiques réducteurs choisis dans le groupe: alcools, aldéhydes, amines, cétones, phénols, oléfines, composés aromatiques et oxygène, soufre, composés azotés et des mélanges de ces produits, caractérisée en ce que le composé organique réducteur est mis en contact avec du dioxyde d'azote sur un catalyseur d'or métallique élémentaire sur support, dans des conditions de température élevée de l'ordre de 250°C à 400°C de façon que le dioxyde d'azote soit réduit en oxyde nitrique et que le composé organique réducteur soit déshydrogéné pour donner un produit à l'état supérieur d'oxydation, le produit déshydrogéne étant ensuite récupéré.

10. Méthode selon la revendication 9, caractérisée en ce que ledit composé réducteur est du cyclohexène et ledit produit obtenu est du benzène.

11. Méthode selon la revendication 9, caractérisé en ce que ledit composé réducteur est une oléfine et ledit produit obtenu est un composé aromatique ou un composé moins saturé.

12. Méthode selon l'une quelconque des revendications 9 à 11, caractérisée, en outre, en ce que après mis en contact du composé réducteur et du dioxyde d'azote sur le catalyseur à l'or, l'oxyde nitrique est oxydé en dioxyde d'azote gazeux puis ce dernier est séparé de l'agent organique réducteur déshydrogéné et le dioxyde d'azote gazeux récupéré est acheminé dans le flux d'arrivée du composé organique réducteur.

13. Dispositif de type réacteur-rédox pour la conversion de composés organiques, caractérisé en ce qu'il comprend, en combinaison:

une colonne chromatographique (10) dans laquelle sont introduits les composés organiques;

des moyens de sortie (12) des produits de ladite colonne,

des moyens (14) connectés aux moyens (12) pour l'introduction d'une source d'oxydes d'azote;

des moyens (13) pour introduire des oxydes d'azote sélectionnés, simultanément à un support inerte, dans ladite source (14) des oxydes d'azote et dans les moyens de sortie (12); et

une chambre de réacteur (15) reliée aux moyens (12) pour recevoir les composés organiques, les oxydes d'azote et le support inerte et contenant un lit (16) de catalyseur constitué par de l'or métallique élémentaire, ladite chambre étant en outre munie de moyens (17) pour chauffer l'or à une température élevée, suffisante pour réduire tous les oxydes d'azote présents en oxyde nitrique et pour convertir les composés organiques présents en constituants de degré d'oxydation supérieur.

14. Dispositif selon la revendication 13, caractérisé en ce que qu'il comporte en outre des moyens (30) d'analyse chimiluminescente, destinés à détecter sélectivement les formes d'oxydation supérieure des composés organiques récupérés de ladite colonne chromatographique (10).

15. Dispositif selon la revendication 14, caractérisé en ce que lesdits moyens d'analyse (30) comprennent des moyens générateurs d'ozone (34) destinés à faire réagir l'ozone produit avec l'oxyde nitrique issu du lit catalytique (16) de façon à former du dioxyde d'azote en un état électronique excité.

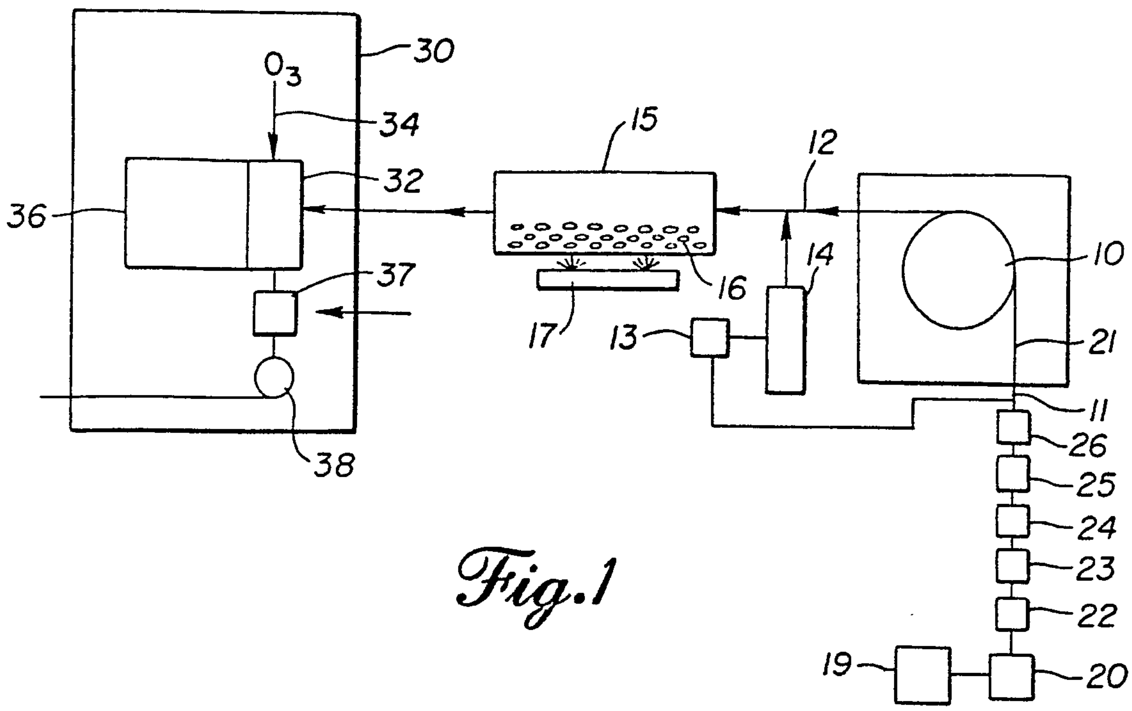

$O_3$
30
34
15
12
32
36
10
14
37
16
17
13
21
11
26
25
24
23
22
38
19
20


Fig.1

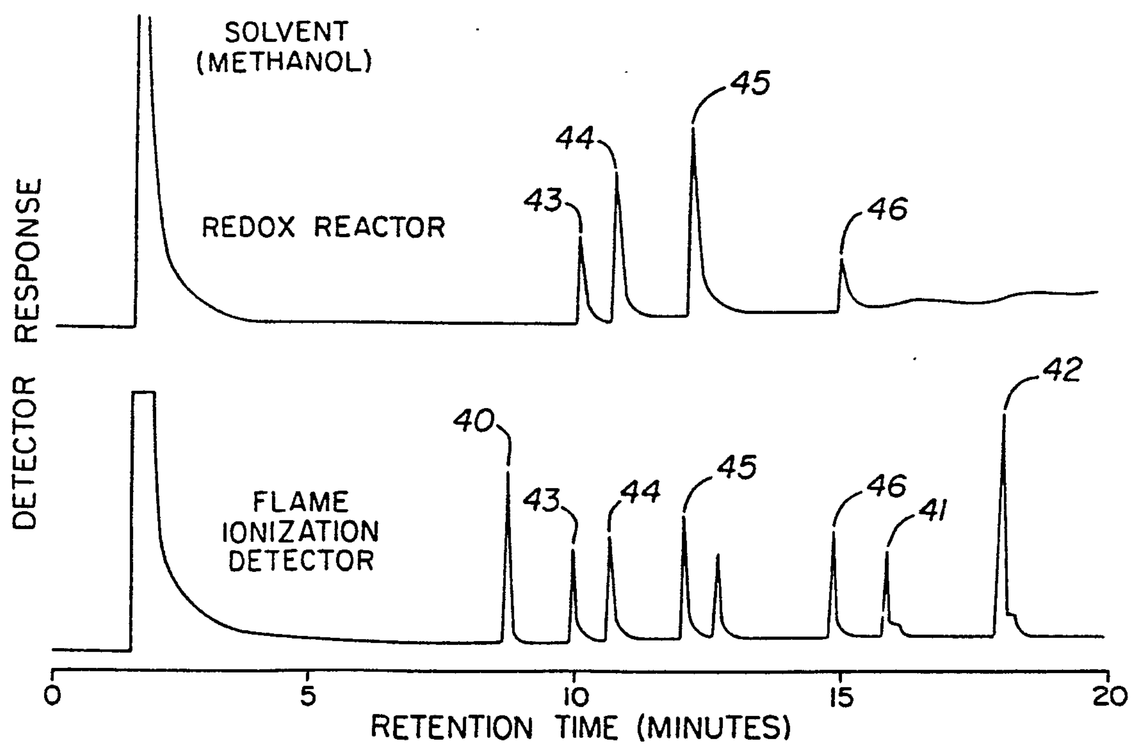

SOLVENT
(METHANOL)
45
44
43
46
REDOX REACTOR
DETECTOR RESPONSE
42
40
43
44
45
46
41
FLAME
IONIZATION
DETECTOR
0
5
10
15
20
RETENTION TIME (MINUTES)


Fig.2